# EUROPEAN PATENT SPECIFICATION

(11) **EP 3 655 018 B1**
(45) Date of publication and mention of the grant of the patent: **26.02.2025**
(21) Application number: 18773681.4
(22) Date of filing: 21.08.2018
(51) Int. Cl.: A61K 38/39, A61K 31/07, A61K 31/11, A61K 31/203, A61K 31/232, A61P 25/00, A61P 27/00, A61P 19/02

(54) **COMPOSITION AND NUTRITIONAL SUPPLEMENT MADE THEREFROM**
ZUSAMMENSETZUNG UND DARAUS HERGESTELLTES NAHRUNGSERGÄNZUNGSMITTEL
COMPOSITION ET COMPLÉMENT NUTRITIONNEL PRODUIT À PARTIR DE CELLE-CI

(30) Priority: 21.08.2017 US 201762547951 P
(43) Date of publication of application: 27.05.2020
(73) Proprietor: Lonza Greenwood LLC, Greenwood, SC 29646 (US)
(72) Inventor: LUGO, Jim, Morristown, NJ 07960 (US); SAIYED, Zainulabedin, Morristown, NJ 07960 (US)
(74) Representative: Greiner, Elisabeth
(86) International application number: PCT/US2018/047267
(87) International publication number: WO 2019/040459

(56) References cited:
- EP-A1- 2 196 099
- EP-B1- 2 196 099
- WO-A1-2011/109586
- WO-A1-2015/017625
- US-A1- 2015 119 335
- US-A1- 2016 199 456
- US-A1- 2016 324 146
- US-B2- 8 703 174
- CHU H ET AL: "Healthcare nutrient solution useful for strengthening brain, contains glucose, caffeine, vitamin A, vitamin B, vitamin D, organic selenium, zinc gluconate, maltodextrin, N-acetylglucosamine, type II collagen, xylitol and menthol", WPI / 2017 CLARIVATE ANALYTICS,, vol. 2015, no. 59, 8 April 2015 (2015-04-08), XP002786590
- CAO R ET AL: "Dietary food composition used for e.g. treating meniscus injury and repairing joints, comprises low molecular weight chondroitin sulfate, collagen peptide, black sesame, wolfberry aqueous extract, oligosaccharide, vitamin and mineral", WPI / 2017 CLARIVATE ANALYTICS,, vol. 2015, no. 13, 24 December 2014 (2014-12-24), XP002786613
- DATABASE WPI Week 201513, Derwent World Patents Index; AN 2015-11726F, XP002786613
- DATABASE WPI Week 201559, Derwent World Patents Index; AN 2015-342567, XP002786590

## Description

### BACKGROUND

In recent years, the use of collagen to treat various conditions has become exceedingly popular. Collagen is a protein that can be found in muscles, bones, skin, blood vessels, and in other parts of the body. There are various different types of collagen depending upon its function and form. For instance, Type I collagen, the most abundant collagen, is made of fibers found in tendons, ligaments, organs and skin. Type II collagen, on the other hand, primarily helps build cartilage, a major structural entity that sits on the surfaces of those bones which comprise articulating joints. Type III collagen is a major component of the extracellular matrix that makes up organs and skin. Type III collagen also forms blood vessels and tissue within the heart.

Collagen can be found in numerous different products including cosmetic creams and body lotions. Collagen is also found in various oral supplements. Collagen production in the body, for instance, tends to slow as a person ages. Thus, collagen supplements have been taken in the past in order to reduce the effects of aging by improving the health of skin and hair.

Collagen has also been found to effectively treat arthritis and other joint pain. For example, U.S. Patent No. 9,066,926 discloses a method of reducing exercise-induced joint pain in mammals by administering to a mammal Type II collagen. This patent also discloses the mechanism of action through which this ingredient operates: oral tolerance. This putative mechanism entails the stimulation of T regulatory cells (Treg), located in gut associated lymphatic tissue, to specifically recognize antigenic determinants (epitopes) on the native collagen protein. Once induced, the Tregs exit the gut area and migrate to the joint space where they stimulate chondrocytes to lay down new Type II collagen thereby enhancing the structural integrity and flexibility of the articulating joint. One such example, for which clinical data has been published is the knee. The '926 patent is incorporated herein by reference.

Various other supplements have been proposed containing bone material. For instance, U.S. Patent No. 9,011,930 is directed to nutritional supplements containing demineralized bone matrix which are believed to act via various bone morphogenetic proteins and other factors that promote bone formation and homeostasis. In this case the principle mode of action is believed to occur via the stimulation of bone forming cells as indicated in the commercial literature for this nutritional ingredient, also known as Cyplexinol^{®}. This supplement may further contain a vitamin and is used to maintain or improve the condition of bones and cartilage.

In addition to treating joint pain, collagen is also known to treat leaky gut syndrome, a condition where toxins are able to pass through a digestive tract. Collagen intake can also boost metabolism, muscle mass, and energy output as well as improve liver and cardiovascular health.

Although collagen can offer various advantages when administered to a human or animal, a need exists for a composition and method that can increase the effectiveness of collagen and/or work in conjunction with collagen to provide synergistic effects such improved efficacy or faster onset of action.

### SUMMARY

In general, the present disclosure is directed to a composition containing the combination of a collagen source and Vitamin A and a carotenoid. The composition of the present disclosure has numerous and diverse applications and uses. The present invention is directed to a nutritional supplement for taking orally comprising a Type II collagen source combined with Vitamin A and a carotenoid, wherein the Type II collagen source comprises an undenatured Type II collagen, wherein the nutritional supplement is in the form of individual dosage vessels, and wherein the individual dosage vessels contain the Type II collagen in an amount of from 20 mg to 100 mg, wherein the carotenoid is lutein. According to the invention, the collagen source comprises an undenatured Type II collagen. The collagen can be obtained from various different sources, such as animal sources and can be supplied as animal parts or can be supplied as a pure protein or as active peptide fragments. The resulting composition can have many different health benefit effects when administered to a human, an animal or other mammal. For instance, the composition is well suited to treating or managing any sort of joint pain, discomfort, or limited performance, to improving eye health, to improving brain health and/or to improving immune health.

In addition to a nutritional supplement, the composition of the present disclosure can also be used as a medicinal composition in order to deliver various health benefits to a human or animal. The medicinal composition, for instance, can be administered orally.

As described above, the composition of the present disclosure generally comprises a collagen source combined with Vitamin A and carotenoid, which is lutein.

The Vitamin A, for instance, can comprise retinol, retinal, retinoic acid or a retinyl ester. For instance, the retinyl ester can comprise a fatty acid retinyl, such as retinyl palmitate. The carotenoid that is particularly well suited for use in the present disclosure is lutein.

When the composition contains undenatured Type II collagen, in combination with Vitamin A, the weight ratio between the collagen and Vitamin A can be from about 10:1 to about 1:5 for the lowest amounts of the Type II collagen source added, and about 5000:1 to about 100:1 for the highest amount of the Type II collagen source added. When the composition contains an undenatured collagen and a carotenoid, the weight ratio between the collagen material and the carotenoid can be from about 2:1 to about 1:1,000 for the lowest amounts of the Type II collagen source added, and about 2,000:1 to about 1:1 for the highest amount of the Type II collagen source added.

According to the invention, the composition is a nutritional supplement in the form of individual dosage vessels. Each dosage vessel can be made from an orally acceptable composition. The dosage vessels, for instance, may comprise capsules, tablets, powders, or the like. Alternatively, the nutritional supplement may be in the form of a syrup or liquid suspension. Alternatively, the composition of the present embodiment can be administered in food or beverage form.

When in the form of individual dosage vessels, each dosage vessel can contain the undenatured Type II collagen in an amount from about 20 mg to about 100 mg. When Vitamin A is present, Vitamin A can be present in each dosage vessel in an amount generally from about 100 micrograms to about 5000 micrograms. When the dosage vessel contains a carotenoid, on the other hand, each dosage vessel can contain the carotenoid in an amount from about 0.5 mg to about 500 mg, such as from about 1 mg to about 150 mg.

Disclosed are methods for treating or managing various ailments. For instance, disclosed is a method for treating or managing joint pain, joint discomfort, or limited joint flexibility in individuals who are not diseased ("healthy subjects"), and whose symptoms are due to participating in normal, daily activities including, but not limited to, strenuous exercise, walking, running, and so on. Further disclosed is a method for treating joint ailments, as described above, that are due to a disease (including arthritis) in humans and other mammals. The method includes administering to the mammal a therapeutically effective amount of the composition or supplement as described above. In one embodiment, the composition contains undenatured Type II collagen in combination with Vitamin A and a carotenoid, which is lutein. The composition, which can be in the form of oral dosage vessels, can be administered to the mammals in an amount sufficient to reduce the joint pain.

Further disclosed is a method for improving eye health. The method includes the step of administering to a mammal the composition or supplement as described above containing the undenatured Type II collagen in combination with Vitamin A and a carotenoid, which is lutein. The composition is administered to the mammal in a therapeutically effective amount sufficient to improve the eye health of the mammal.

Further disclosed is a method for improving brain health. The method includes the step of administering to a mammal the composition or supplement as described above containing the undenatured Type II collagen in combination with Vitamin A and a carotenoid, which is lutein. The composition is administered to the mammal in a therapeutically effective amount sufficient to improve the brain health of the mammal. The brain/cognitive health benefits provided by the composition can vary depending upon the particular application and the desired results. For instance, the composition can improve memory, verbal fluency, and/or various other cognitive functions.

### DEFINITIONS

The term "therapeutically effective amount" as used herein, with respect to the composition described above, shall mean that dosage, or amount of composition, that provides the specific pharmacological or nutritional response for which the composition (containing the collagen source combined with Vitamin A, a carotenoid, or mixtures thereof), is administered or delivered to subjects in need of such treatment. It is emphasized that 'therapeutically effective amount,' administered to a particular subject in a particular instance will not always be effective in treating the ailments or otherwise improving health described herein, even though such dosage is deemed a "therapeutically effective amount" by those skilled in the art. Specific subjects may, in fact, be "refractory" to a "therapeutically effective amount". For example, a refractory subject may have a low bioavailability such that clinical efficacy is not obtainable. It is to be further understood that the composition, or supplement, in particular instances, can be measured as oral dosages, or with reference to ingredient levels that can be measured in blood. In other embodiments, dosages can be measured in amounts applied to the skin when the composition is contained with a topical formulation.

The term "delivering" or "administering" as used herein, refers to any route for providing the composition, or a nutraceutical, to a subject that is accepted as standard by the medical community and as regulated by law. For example, the present invention addresses routes of delivering or administering that include oral ingestion plus any other suitable route of delivery including, but not limited to, transdermal, intravenous, intraperitoneal, intramuscular, topical or subcutaneous.

The term "encapsulate", "encapsulated", or "encapsulating" refers to any compound that is completely surrounded by a protective material. For example, a compound may become encapsulated by a population of nanoemulsion particle formation during microfluidization.

The term "nutraceutical" refers to any compound added to a dietary source (i.e., for example, a fortified food or a dietary supplement) that provides health or medical benefits in addition to its basic nutritional value.

Other features and aspects of the present disclosure are discussed in greater detail below.

### DETAILED DESCRIPTION

The present invention is directed to a nutritional supplement for taking orally comprising a Type II collagen source combined with Vitamin A and a carotenoid, wherein the Type II collagen source comprises an undenatured Type II collagen, wherein the nutritional supplement is in the form of individual dosage vessels, and wherein the individual dosage vessels contain the Type II collagen in an amount of from 20 mg to 100 mg, wherein the carotenoid is lutein. Of particular advantage, the composition can be formulated so as to treat or improve the health of many different organ systems within the body. In fact, the composition of the present disclosure can provide multiple health benefits simultaneously.

For instance, the composition of the present disclosure is particularly formulated to improve joint health. For instance, the composition can be used to treat arthritis or non-arthritic joint pain, joint discomfort, or lack of joint flexibility. The composition of the present disclosure can also be used to improve eye health and treat various eye ailments. In addition, the composition of the present disclosure can improve immune health or brain health.

In general, the composition of the present disclosure contains undenatured Type II collagen in combination with another dietary or nutraceutical agent that, in one embodiment, can work synergistically with the collagen. For example, in one embodiment, the dietary or nutraceutical agent can increase the effectiveness of the collagen. In addition, or alternatively, the combination of collagen with the dietary or nutraceutical agent, or ingredient, can provide a platform for treating more than one aliment or otherwise improving the health of multiple organ systems within the body.

According to the invention, the undenatured Type II collagen is combined with both Vitamin A and a carotenoid, which is lutein. The nutritional supplement formulated in accordance with the present disclosure can be administered in an oral form. In one embodiment, the nutritional supplement is formed into a dosage vessel, such as a tablet or capsule that can be taken orally. In addition, the nutritional supplement can be incorporated into beverages, foods and dietary supplements. The nutritional supplement, for instance, can be manufactured in the form of capsules, tablets, gummy chewables, edible films, lozenges, powders, liquid suspensions, syrups, and the like. In one embodiment, the nutritional supplement is encapsulated by a protective material in forming a capsule or tablet.

The composition can be taken regularly (i.e. daily or weekly) or can be taken for a temporary period of time in order to treat or manage a particular condition. As indicated above, the composition contains an undenatured Type II collagen. Type II collagen for use in the present disclosure can be obtained from any suitable source. For instance, the collagen can be derived from a variety of mammalian sources, avian sources, or can be obtained from various fish species or a combination thereof. For instance, the collagen can be obtained from salmon, shark, poultry, porcine, egg shells, turkey cartilage, bovine cartilage, and the like. In one embodiment, for instance, the Type II collagen can be obtained as disclosed in U.S. Patent No. 7,083,820 to Schilling. For example, undenatured Type II collagen is available commercially as UC-II^{®} from InterHealth Nutraceuticals. UC-II^{®} is a natural ingredient that contains a glycosylated, undenatured Type II collagen. The collagen source can also comprise a pure protein or active peptide fragments. In one embodiment, the collagen source can be free of any bone or bone material. In other embodiments, the collagen source can be free of any transforming growth factors (TGFs), bone morphogenetic proteins (BMPs), or both. In still another embodiment, the collagen source comprises Type II collagen and is completely free of any Type I collagen.

In preparing animal tissue for oral administration, in one embodiment, the Type II collagen containing tissue can be first dissected free of surrounding tissues and diced or otherwise comminuted into particles. The particulate, or milled, cartilage can be sterilized by means which do not affect or denature the structure of a major portion of the Type II collagen in the tissue and formed into doses containing therapeutically effective levels of undenatured Type II collagen, said levels being generally in the amount of at least about 0.01 gram and preferably from about 0.02 to about 0.5 grams of animal tissue in a dose. Being a natural product some variation from sample to sample is to be expected. These variations can be minimized by blending after comminution. The blending can be aided by analytical techniques which allow the measurement of the amount of undenatured Type II collagen and other constituents.

These measurements will allow blending of batches to obtain uniformity and in some cases to modify potency by increasing certain antigenic epitopes via the mixing of cartilage from different sources. The optimum dosage may vary.

In accordance with the present disclosure, the undenatured Type II collagen is combined with Vitamin A and a carotenoid, which is lutein. Vitamin A refers to a group of organic compounds that include various retinoids. For instance, Vitamin A includes retinol, which is also known as Vitamin A₁. Other Vitamin A compounds include all different forms of retinoic acid, such as tretinoin, which is also known as all-trans retinoic acid. Other Vitamin A compounds include retinal and retinyl esters. Retinyl esters include fatty acid esters of retinyl. For example, one example is retinyl palmitate. Another Vitamin A compound is retinyl acetate. Retinyl acetate and retinyl palmitate are typically referred to as provitamins of Vitamin A and are converted to retinol in the small intestine.

Vitamin A intake in animals and humans can improve immunity health, vision health, reproductive health, brain and cognitive health, and improve cellular communication. In improving immune health, it is believed that retinoic acid regulates dendritic cell differentiation, migration, and antigen presenting capacity, particularly in gut associated lymphatic tissue. Retinoic acid also maintains homeostasis by working with TGF-beta to promote Treg cell induction from naïve T cells. Retinoic acid can also be important in the maintenance of mucosal integrity.

Alternatively, or in addition to Vitamin A, the composition of the present disclosure may contain one or more carotenoids (that are separate from the Vitamin A species described above). Certain carotenoids are known to be effective antioxidant agents which can protect organisms from the adverse effects caused by active oxygen species, e.g., free radicals and the like. Carotenoid intake can also lower the chance of chronic diseases, such as heart disease and various cancers. It is believed that inclusion of a carotenoid in the nutritional supplement can improve the immune system of the human or animal. Certain carotenoids, such as lutein, can also provide brain and cognitive health benefits. For instance, the carotenoid can improve memory, improve verbal fluency, or otherwise have beneficial effects on cognitive functions. Of particular advantage, it is believed that a carotenoid can work effectively with the undenatured Type II collagen to increase the effectiveness of the overall composition.

Carotenoid compounds are a class of tetraterpenoids which contain long polyene chains. Carotenoids include xanthophylls such as lutein and zeaxanthin, and carotenes, such as beta-carotene, alpha-carotene, zeto-carotene, and lycopene and related molecules, including 1-HO-3',4'-didehydrolycopene, 3, 1'-(HO)2-gamma-carotene, 1,1'-(HO)2-3, 4, 3',4'-tetradehydrolycopene, 1, 1'-(HO)2-3, 4-didehydrolycopene.

Other carotenoid compounds include hydrocarbons, such as lycopersene (7,8,11,12,15,7',8',11',12',15'-decahydro-γ,γ-carotene), phytofluene, hexahydrolycopene (15-cis-7,8,11,12,7',8'-hexahydro-γ,γ-carotene), torulene (3',4'-didehydro-β,γ-carotene) and α-zeacarotene (7',8'-dihydro-ε,γ-carotene); alcohols, such as alloxanthin, cynthiaxanthin, pectenoxanthin, cryptomonaxanthin, ((3r,3'r)-7,8,7',8'-tetradehydro-β,β-carotene-3,3'-diol), crustaxanthin (β,-carotene-3,4,3',4'-tetrol), gazaniaxanthin ((3r)-5'-cis-β,γ-caroten-3-ol), oh-chlorobactene (1',2'-dihydro-f,γ-caroten-1'-ol), loroxanthin (β,ε-carotene-3,19,3'-triol), lycoxanthin (γ,γ-caroten-16-ol), rhodopin (1,2-dihydro-γ,γ-caroten-1-ol), rhodopinol (aka warmingol; 13-cis-1,2-dihydro-γ,γ-carotene-1,20-diol), saproxanthin (3',4'-didehydro-1',2'-dihydro-β,γ-carotene-3,1'-diol) and zeaxanthin; glycosides, such as oscillaxanthin (2,2'-bis(β-I-rhamnopyranosyloxy)-3,4,3',4'-tetradehydro-1,2,1',2'-tetrahydro-γ,γ-carotene-1,1'-diol), and phleixanthophyll (1'-(β-d-glucopyranosyloxy)-3',4'-didehydro-1',2'-dihydro-β,γ-caroten-2'-ol); ethers, such as rhodovibrin (1'-methoxy-3',4'-didehydro-1,2,1',2'-tetrahydro-γ,γ-caroten-1-ol) and spheroidene (1-methoxy-3,4-didehydro-1,2,7',8'-tetrahydro-γ,γ-carotene), epoxides, such as diadinoxanthin (5,6-epoxy-7',8'-didehydro-5, 6-dihydro-carotene-3,3-diol), luteoxanthin (5,6: 5',8'-diepoxy-5,6,5',8'-tetrahydro-β,β-carotene-3,3'-diol), mutatoxanthin, citroxanthin, zeaxanthin (furanoxide 5,8-epoxy-5,8-dihydro-β,β-carotene-3,3'-diol), neochrome (5',8'-epoxy-6,7-didehydro-5,6,5',8'-tetrahydro-β,β-carotene-3,5,3'-triol), foliachrome, trollichrome, and vaucheriaxanthin (5',6'-epoxy-6,7-didehydro-5,6,5',6'-tetrahydro-β,β-carotene-3,5,19,3'-tetrol); aldehydes, such as rhodopinal, wamingone (13-cis-1-hydroxy-1,2-dihydro-γ,γ-caroten-20-al), torularhodinaldehyde (3',4'-didehydro-β,γ-caroten-16'-al); acids and acid esters, such as torularhodin (3',4'-didehydro-β,γ-caroten-16'-oic acid) and torularhodin methyl ester (methyl 3',4'-didehydro-β,γ-caroten-16'-oate); ketones, such as astaxanthin, canthaxanthin (aka aphanicin), chlorellaxanthin (β,β-carotene-4,4'-dione), capsanthin ((3r,3's,5'r)-3,3'-dihydroxy-β,κ-caroten-6'-one), capsorubin ((3s,5r,3's,5'r)-3,3'-dihydroxy-κ,κ-carotene-6,6'-dione), cryptocapsin ((3'r,5'r)-3'-hydroxy-β,κ-caroten-6'-one), 2,2'-diketospirilloxanthin (1,1'-dimethoxy-3,4,3',4'-tetradehydro-1,2,1',2'-tetrahydro-γ,γ-carotene-2,2'-dione), flexixanthin (3,1'-dihydroxy-3',4'-didehydro-1',2'-dihydro-β,γ-caroten-4-one), 3-oh-canthaxanthin (aka adonirubin; aka phoenicoxanthin; 3-hydroxy-β,β-carotene-4,4'-dione), hydroxyspheriodenone (1'-hydroxy-1-methoxy-3, 4-didehydro-1,2,1',2',7',8'-hexahydro-γ,γ-caroten-2-one), okenone (1'-methoxy-1',2'-dihydro-c,γ-caroten-4'-one), pectenolone (3,3'-dihydroxy-7',8'-didehydro-β,β-caroten-4-one), phoeniconone (aka dehydroadonirubin; 3-hydroxy-2,3-didehydro-β,β-carotene-4,4'-dione), phoenicopterone (β,ε-caroten-4-one), rubixanthone (3-hydroxy-β,γ-caroten-4'-one), siphonaxanthin (3,19,3'-trihydroxy-7,8-dihydro-β,ε-caroten-8-one); esters of alcohols, such as astacein (3,3'-bispalmitoyloxy-2,3,2',3'-tetradehydro-β,β-carotene-4,4'-dione or 3,3'-dihydroxy-2,3,2',3'-tetradehydro-β,β-carotene-4,4'-dione dipalmitate), fucoxanthin (3'-acetoxy-5,6-epoxy-3,5'-dihydroxy-6',7'-didehydro-5,6,7,8,5',6'-hexahydro-β,β-caroten-8-one), isofucoxanthin (3'-acetoxy-3,5,5'-trihydroxy-6',7'-didehydro-5,8,5',6'-tetrahydro-β,β-caroten-8-one), physalien, zeaxanthin dipalmitate ((3r,3'r)-3,3'-bispalmitoyloxy-β,β-carotene or (3r,3'r)-β,β-carotene-3,3'-diol dipalmitate) and siphonein (3,3'-dihydroxy-19-lauroyloxy-7,8-dihydro-β,ε-caroten-8-one or 3,19,3'-trihydroxy-7,8-dihydro-β,ε-caroten-8-one 19-laurate); apo carotenoids, such as β-apo-2'-carotenal (3',4'-didehydro-2'-apo-b-caroten-2'-al), apo-2-lycopenal, apo-6'-lycopenal (6'-apo-y-caroten-6'-al), azafrinaldehyde (5,6-dihydroxy-5,6-dihydro-10'-apo-β-caroten-10'-al), bixin (6'-methyl hydrogen 9'-cis-6,6'-diapocarotene-6,6'-dioate), citranaxanthin (5',6'-dihydro-5'-apo-β-caroten-6'-one or 5',6'-dihydro-5'-apo-18'-nor-β-caroten-6'-one or 6'-methyl-6'-apo-β-caroten-6'-one), crocetin (8,8'-diapo-8,8'-carotenedioic acid), crocetinsemialdehyde (8'-oxo-8,8'-diapo-8-carotenoic acid), crocin (digentiobiosyl 8,8'-diapo-8,8'-carotenedioate), hopkinsiaxanthin (3-hydroxy-7,8-didehydro-7',8'-dihydro-7'-apo-b-carotene-4,8'-dione or 3-hydroxy-8'-methyl-7,8-didehydro-8'-apo-b-carotene-4,8'-dione), methyl apo-6'-lycopenoate (methyl 6'-apo-y-caroten-6'-oate), paracentrone (3,5-dihydroxy-6,7-didehydro-5,6,7',8'-tetrahydro-7'-apo-b-caroten-8'-one or 3,5-dihydroxy-8'-methyl-6,7-didehydro-5,6-dihydro-8'-apo-b-caroten-8'-one) and sintaxanthin (7',8'-dihydro-7'-apo-b-caroten-8'-one or 8'-methyl-8'-apo-b-caroten-8'-one); nor and seco carotenoids, such as actinioerythrin (3,3'-bisacyloxy-2,2'-dinor-b,b-carotene-4,4'-dione), β-carotenone (5,6:5',6'-diseco-b,b-carotene-5,6,5',6'-tetrone), peridinin (3'-acetoxy-5,6-epoxy-3,5'-dihydroxy-6',7'-didehydro-5,6,5',6'-tetrahydro-12',13',20'-trinor-b,b-caroten-19,11-olide), pyrrhoxanthininol (5,6-epoxy-3,3'-dihydroxy-7',8'-didehydro-5,6-dihydro-12',13',20'-trinor-b,b-caroten-19,11-olide), semi-α-carotenone (5,6-seco-b,e-carotene-5,6-dione), semi-β-carotenone (5,6-seco-b,b-carotene-5,6-dione or 5',6'-seco-b,b-carotene-5',6'-dione) and triphasiaxanthin (3-hydroxysemi-b-carotenone 3'-hydroxy-5,6-seco-b,b-carotene-5,6-dione or 3-hydroxy-5',6'-seco-b,b-carotene-5',6'-dione); retro carotenoids and retro apo carotenoids, such as eschscholtzxanthin (4',5'-didehydro-4,5'-retro-b,b-carotene-3,3'-diol), eschscholtzxanthone (3'-hydroxy-4',5'-didehydro-4,5'-retro-b,b-caroten-3-one), rhodoxanthin (4',5'-didehydro-4,5'-retro-b,b-carotene-3,3'-dione) and tangeraxanthin (3-hydroxy-5'-methyl-4,5'-retro-5'-apo-b-caroten-5'-one or 3-hydroxy-4,5'-retro-5'-apo-b-caroten-5'-one); and higher carotenoids, such as nonaprenoxanthin (2-(4-hydroxy-3-methyl-2-butenyl)-7',8',11',12'-tetrahydro-e,y-carotene), decaprenoxanthin (2,2'-bis (4-hydroxy-3-methyl-2-butenyl)-e,e-carotene), c.p. 450 (2-[4-hydroxy-3-(hydroxymethyl)-2-butenyl]-2'-(3-methyl-2-butenyl)-b,b-carotene), c.p. 473 (2'-(4-hydroxy-3-methyl-2-butenyl)-2-(3-methyl-2-butenyl)-3',4'-didehydro-1',2'-dihydro-b,y-caroten-1'-ol) and bacterioruberin (2,2'-bis(3-hydroxy-3-methylbutyl)-3,4,3',4'-tetradehydro-1,2,1',2'-tetrahydro-y,y-carotene-1,1'-dio).

Carotenoid compounds for use as described herein may be natural i.e. obtained from a natural source, for example, extracted from a plant, such as a tomato, melon, or marigold, etc. A range of methods for extracting, concentrating and/or purifying carotenoids from plants are known in the art. For example, solvent extraction using ethanol, DMSO, ethyl acetate, hexane, acetone, soya or other vegetable oil, or non-vegetable oils may be employed. A carotenoid compound may be isolated free, or substantially free, of other molecules found in its natural source or environment. The use of such extraction and purification methods are known to individuals appropriately skilled in the art.

Carotenoid compounds for use as described herein may be synthetic i.e. produced by artificial means, for example, by chemical synthesis or fermentation. A range of methods for chemical synthesis of carotenoids are known in the art.

The carotenoid contained in the composition is a carotenoid with antioxidant activities, which is lutein. The above carotenoids, especially lutein, have been found to be well matched with a Type II collagen. For instance, Type II collagen can affect T-reg cells to produce anti-inflammatory cytokines that convert chondrocyte physiology from matrix proteins break down to the creation and deposition of matrix proteins. Various carotenoids, on the other hand, such as lutein are powerful antioxidants. Lutein may activate the Nrf2 pathway providing cytoprotective activity and reduced inflammation. In the case of lutein, it has been shown that it can reduce monosodium iodoacetate (MIA) induced oxidative stress in primary chondrocytes, as well as mitigate the symptoms of OA induced by MIA in rats. In this manner, the carotenoid can synergistically operate with the Type II collagen in the nutritional supplement to provide overall better efficacy. For example, in one embodiment, it is believed that the combination of a Type II collagen and a carotenoid with antioxidant activity can provide an overall better composition for treating joint pain resulting from participation in normal, everyday activities or from arthritis.

Carotenoids described herein can also include carotenoid precursors that release carotenoids in the body due to metabolic processes or the like. For instance, the carotenoid may comprise a precursor or compound that results in the synthesis of any of the above described carotenoids, such as the carotenoids with antioxidant activities. The carotenoid described herein can comprise a precursor that converts into lycopene once ingested or otherwise incorporated into the body of a patient.

The amount of undenatured Type II collagen and the amount of Vitamin A and lutein in a dose consumed at any given time will vary with the purpose of the consumption, the severity of symptoms, as well as the condition, age, weight, medical history and general physical characteristics of the subject (human or animal) to be treated. Consequently, the dosages, the frequency and time period over which the dosages are administered can vary widely. The nutritional supplement of the present disclosure can be combined with other digestible ingredients such as in the form of aqueous dispersions, such as milk, or in combination with other protein-rich substances, sugars and starches. In one embodiment, the nutritional supplement may be administered directly as a comminuted solid as in an encapsulated comminuted solid, such as a compression and formed pill, as well as a slurry with or without other digestible compositions such as, for example, foodstuffs.

In one embodiment, when the nutritional supplement contains undenatured Type II collagen combined with Vitamin A, the weight ratio between the Type II collagen and the Vitamin A can generally be from about 100:1 to about 20:1. For instance, the weight ratio between the collagen and the Vitamin A can be from about 80:1 to about 30:1, such as from about 60:1 to about 35:1.

When the undenatured Type II collagen is combined with a carotenoid, which is lutein, on the other hand, the weight ratio between the collagen and the carotenoid can be from about 40:1 to about 1:10, such as from about 20:1 to about 1:4. In one embodiment, the weight ratio between the collagen and said carotenoid can be from about 15:1 to about 1:4.

According to the invention, the composition or nutritional supplement of the present disclosure is formed into individual dosage vessels that are intended to be taken orally by a human or animal. The dosage vessel, for instance, may comprise a tablet, capsule or other delivery vehicle. According to the invention, the undenatured Type II collagen can be present in each dosage vessel generally in an amount from 20 milligram to 100 milligrams. For instance, the Type II collagen can be present in each dosage vessel in an amount greater than 20 milligrams, such as in an amount greater than about 25 milligrams, such as in an amount greater than about 30 milligrams. The amount of Type II collagen present in each dosage vessel can generally be less than 100 milligrams, such as less than about 80 milligrams, such as less than about 70 milligrams, such as less than about 60 milligrams.

When the Type II collagen is combined with Vitamin A, such as retinol or its esterified form, the Vitamin A can be present in each dosage vessel in an amount greater than about 100 micrograms, such as greater than about 200 micrograms, such as greater than about 300 micrograms, such as greater than about 400 micrograms, such as greater than about 500 micrograms, such as greater than about 600 micrograms, such as greater than about 700 micrograms. The Vitamin A can be present in each dosage vessel in an amount less than about 5000 micrograms, such as less than about 3000 micrograms, such as less than about 2000 micrograms, such as less than about 1000 micrograms, such as less than about 900 micrograms.

When the Type II collagen is combined with a carotenoid, the carotenoid can be present in the dosage vessel in an amount generally greater than about 0.5 milligrams, such as greater than about 1 milligram, such as greater than about 2 milligrams, such as greater than about 2.5 milligrams, such as greater than about 5 milligrams, such as greater than about 10 milligrams, such as greater than about 15 milligrams, such as greater than about 20 milligrams, such as greater than about 25 milligrams, such as greater than about 30 milligrams, such as greater than about 35 milligrams, such as greater than about 40 milligrams. The carotenoid is generally present in an amount less than about 5000 milligrams, such as less than about 3000 milligrams, such as less than about 1000 milligrams, such as less than about 800 milligrams, such as less than about 600 milligrams, such as less than about 400 milligrams, such as less than about 200 milligrams, such as less than about 100 milligrams, such as less than about 90 milligrams, such as less than about 80 milligrams, such as less than about 70 milligrams, such as less than about 60 milligrams. According to the invention, the carotenoid is lutein and can be present in each individual dosage vessel in an amount from about 1 milligram to about 140 milligrams, such as from about 2.5 milligrams to about 60 milligrams.

The above dosage vessels can be taken regularly over a substantial period of time. For instance, the dosage vessels can be taken daily, can be taken every two to five days, can be taken weekly, or can be taken every two weeks.

As described above, according to the invention, the composition of the present disclosure can be formulated as a nutritional supplement that is taken orally. It should be understood, however, that the composition of the present disclosure can be administered to a human or animal using any suitable method.

The nutritional supplement as defined above can be used for administering the nutritional supplement to a human or animal. For example, it is useful for treating joint pain including arthritis . The nutritional supplement is administered to the mammal in a therapeutically effective amount so as to reduce joint pain or to otherwise treat join pain. For instance, the reduction in joint pain is evidenced by improvements in joint (e.g., knee, elbow, etc.) range motion, an increase in levels of activity, or any activity that depends on having functional joints.

**The** present nutritional supplement as defined above may be useful for treating a mammal in order to improve immune health, eye health and/or brain health, and may improve the eye health, the immune health and the brain health of the mammal.

## Claims

1. A nutritional supplement for taking orally comprising:
a Type II collagen source combined with Vitamin A and a carotenoid, wherein the Type II collagen source comprises an undenatured Type II collagen, wherein the nutritional supplement is in the form of individual dosage vessels, and wherein the individual dosage vessels contain the Type II collagen in an amount of from 20 mg to 100 mg, wherein the carotenoid is lutein.

2. A nutritional supplement as defined in any of the preceding claims, wherein the individual dosage vessels comprise tablets, capsules, or powders.

3. A nutritional supplement as defined in any of the preceding claims, the Vitamin A comprising retinol or a retinyl ester.

4. A nutritional supplement as defined in claim 1, wherein the individual dosage vessels contain from about 20 mg to about 100 mg of the Type II collagen.

5. A nutritional supplement as defined in claim 2 or 4, the Vitamin A being present in each dosage vessel in an amount from about 100 micrograms to about 5000 micrograms.

6. A nutritional supplement as defined in claim 2 or 4, the carotenoid being present in each dosage vessel in an amount from about 0.5 mg to about 1000 mg, such as from about 1 mg to about 150 mg.

7. A nutritional supplement as defined in claim 1, the Type II collagen source being present in the composition in relation to the carotenoid in a weight ratio of from about 2:1 to about 1:1,000 for the lowest amounts of the Type II collagen source added, and about 2,000:1 to about 1:1 for the highest amount of the Type II collagen source added.

8. A nutritional supplement as defined in claim 1, the Type II collagen source being present in the composition in relation to the Vitamin A at a weight ratio of from about 100:1 to about 20:1.

## Patentansprüche

1. Nahrungsergänzungsmittel zur oralen Einnahme, umfassend:
eine Typ-II-Kollagenquelle, die mit Vitamin A und einem Carotinoid kombiniert ist, wobei die Typ-II-Kollagenquelle ein nicht denaturiertes Typ-II-Kollagen umfasst, wobei das Nahrungsergänzungsmittel in Form von einzelnen Dosierungsbehältern vorliegt und wobei die einzelnen Dosierungsbehälter das Typ-II-Kollagen in einer Menge von 20 mg bis 100 mg enthalten, wobei das Carotinoid Lutein ist.

2. Nahrungsergänzungsmittel nach einem der vorhergehenden Ansprüche, wobei die einzelnen Dosierungsbehälter Tabletten, Kapseln oder Pulver umfassen.

3. Nahrungsergänzungsmittel nach einem der vorhergehenden Ansprüche, wobei das Vitamin A Retinol oder einen Retinylester umfasst.

4. Nahrungsergänzungsmittel nach Anspruch 1, wobei die einzelnen Dosierungsbehälter etwa 20 mg bis etwa 100 mg des Typ-II-Kollagens enthalten.

5. Nahrungsergänzungsmittel nach Anspruch 2 oder 4, wobei das Vitamin A in jedem Dosierungsbehälter in einer Menge von etwa 100 Mikrogramm bis etwa 5.000 Mikrogramm vorhanden ist.

6. Nahrungsergänzungsmittel nach Anspruch 2 oder 4, wobei das Carotinoid in jedem Dosierungsbehälter in einer Menge von etwa 0,5 mg bis etwa 1.000 mg, wie beispielsweise von etwa 1 mg bis etwa 150 mg, vorhanden ist.

7. Nahrungsergänzungsmittel nach Anspruch 1, wobei die Typ-II-Kollagenquelle in der Zusammensetzung im Verhältnis zum Carotinoid in einem Gewichtsverhältnis von etwa 2:1 bis etwa 1:1.000 für die geringsten Mengen der zugesetzten Typ-II-Kollagenquelle und etwa 2.000:1 bis etwa 1:1 für die höchste Menge der zugesetzten Typ-II-Kollagenquelle vorhanden ist.

8. Nahrungsergänzungsmittel nach Anspruch 1, wobei die Typ-II-Kollagenquelle in der Zusammensetzung im Verhältnis zum Vitamin A in einem Gewichtsverhältnis von etwa 100:1 bis etwa 20:1 vorhanden ist.

## Revendications

1. Supplément nutritionnel à prendre par voie orale comprenant :
une source de collagène de type II combinée à de la vitamine A et à un caroténoïde, dans lequel la source de collagène de type II comprend un collagène de type Il non dénaturé, dans lequel le supplément nutritionnel se présente sous la forme de récipients de dosage individuels, et dans lequel les récipients de dosage individuels contiennent le collagène de type II dans une quantité comprise entre 20 mg et 100 mg, dans lequel le caroténoïde est la lutéine.

2. Supplément nutritionnel tel que défini selon l'une quelconque des revendications précédentes, dans lequel les récipients de dosage individuels comprennent des comprimés, des gélules ou des poudres.

3. Supplément nutritionnel tel que défini selon l'une quelconque des revendications précédentes, la vitamine A comprenant du rétinol ou un ester de rétinyle.

4. Supplément nutritionnel tel que défini selon la revendication 1, dans lequel les récipients de dosage individuels contiennent entre environ 20 mg et environ 100 mg de collagène de type II.

5. Supplément nutritionnel tel que défini selon la revendication 2 ou la revendication 4, la vitamine A étant présente dans chaque récipient de dosage en une quantité comprise entre environ 100 microgrammes et environ 5 000 microgrammes.

6. Supplément nutritionnel tel que défini selon la revendication 2 ou la revendication 4, le caroténoïde étant présent dans chaque récipient de dosage en une quantité comprise entre environ 0,5 mg et environ 1 000 mg, telle que comprise entre environ 1 mg et environ 150 mg.

7. Supplément nutritionnel tel que défini selon la revendication 1, la source de collagène de type Il étant présente dans la composition par rapport au caroténoïde dans un rapport de poids compris entre environ 2:1 et environ 1:1 000 pour les quantités les plus faibles de la source de collagène de type Il ajoutée, et entre environ 2 000:1 et environ 1:1 pour la quantité la plus élevée de la source de collagène de type Il ajoutée.

8. Supplément nutritionnel tel que défini selon la revendication 1, la source de collagène de type Il étant présente dans la composition par rapport à la vitamine A dans un rapport de poids compris entre environ 100:1 et environ 20:1.
